Europäisches Patentamt

European Patent Office

Office européen des brevets

(19)

(11) **EP 1 598 008 A1**

(12) **EUROPEAN PATENT APPLICATION**

(43) Date of publication:
**23.11.2005 Bulletin 2005/47**

(51) Int Cl.⁷: **A61B 5/11**, A61B 5/145

(21) Application number: **05010796.0**

(22) Date of filing: **18.05.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **18.05.2004 JP 2004147124**

(71) Applicant: **Hitachi, Ltd.
Tokyo 100-8280 (JP)**

(72) Inventors:
• **Yamamoto, Tsuyoshi c/o Intellectual Prop. Office**
**6-1, Marunouchi 1-chome Tokyo 100-8220 (JP)**
• **Kandori, Akihiko c/o Intellectual Prop. Office**
**6-1, Marunouchi 1-chome Tokyo 100-8220 (JP)**

(74) Representative: **Strehl Schübel-Hopf & Partner
Maximilianstrasse 54
80538 München (DE)**

(54) **Living body measurement system by light, image display methodology and program**

(57)     A method is provided which permits a reproduction time to verify a measurement image in a living body light measurement to be shortened, an effective performance check to be made, and periods containing noise and periods where tasks are not performed correctly to be eliminated in a short time. A moving image of a subject is photographed in a first trial period (rest period when the subject is at rest and task period when a task is given to the subject) and a second trial period (rest period when the subject is at rest and task period when a task is given to the subject), and a moving image in the first trial period and a moving image in the second trial period are displayed effectively simultaneously. The performance check in each trial period can be performed efficiently, and the moving image reproduction time can be shortened. Further, periods containing noise and periods where tasks are not performed correctly can be eliminated in a shorter time than in the prior art.

FIG. 1

**Description**

CLAIM OF PRIORITY

The present application claims priority from Japanese application JP 2004-147124 filed on May 18, 2004, the content of which is hereby underlined incorporated by reference into this application.

FIELD OF THE INVENTION

A method of displaying the result of imaging a subject's behavior in a living body measurement system by light which measures a metabolite concentration change inside the subject using light, and images the measurement result.

BACKGROUND OF THE INVENTION

A technique (hereafter, referred to as a living body light measurement method) which measures and images a metabolite concentration change in a living body using light such as near-infrared light which has high living body tissue permeability, is described in Patent document 1 and Patent document 2, and its validity in brain function measurement is described in Non-patent document 1 and Non-patent document 2. An outline of this technique is described below.

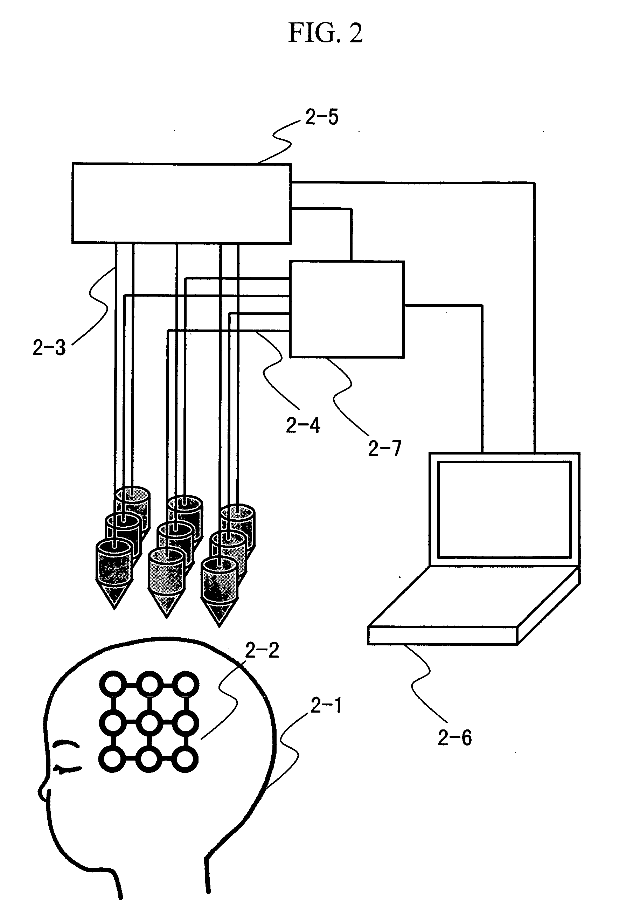

FIG. 2 shows the layout of a system which implements this living body light measurement method. A subject 2-1 can be measured by fitting him with a helmet (2-2) to which measurement optical fibers, described later, can be attached. These fibers comprise illumination optical fibers (2-3) which irradiate light from a light source onto the subject, and detection optical fibers (2-4) which condense light that has passed through the living body, and transmit it to a detector. In the present aspect of the invention, fibers of the first type are grouped in sets of five, and fibers of the second type are grouped in sets of four, respectively. Since the tips of these illumination optical fibers and detection optical fibers are in contact with the skull of the subject (2-1), the subject is not damaged but can be measured in an everyday living environment. The illumination optical fibers and detection optical fibers are arranged at intervals of about 30 mm, for example at the intersections of a grid. The illumination optical fibers are connected to a light source array (2-5). This light source array comprises plural light sources such as a semiconductor laser and a light emitting diode. The number of light sources is equivalent to the product obtained by multiplying the number of illumination optical fibers by the number of materials to be measured. The intensity of each light source is controlled by a computer indicated by 2-6.

The light source array has an optical combiner which can direct light illuminated from plural light sources having different wavelengths to each illumination optical fiber. The detection optical fibers which detect the light that has dispersed within the subject are connected to a detector array (2-7) comprising a light detector such as a photo-diode or a photo-multiplier. The light transmitted to this detector array is converted into an electric signal, and light intensity information is then transferred to the computer indicated by 2-6 in real time. This computer, in addition to controlling the intensity of the light source mentioned above, also has a function to calculate the concentration change of a living body metabolite from a change of the transmitted light intensity, and a function to display the concentration change of the living body metabolite as a time course or an image from the computation result.

In general, the living body metabolites used as measurement candidates are oxygenated hemoglobin (Hb) and deoxygenated Hb, and spectral measurements are performed using laser light of plural wavelengths (e.g., 780 nm and 830 nm).

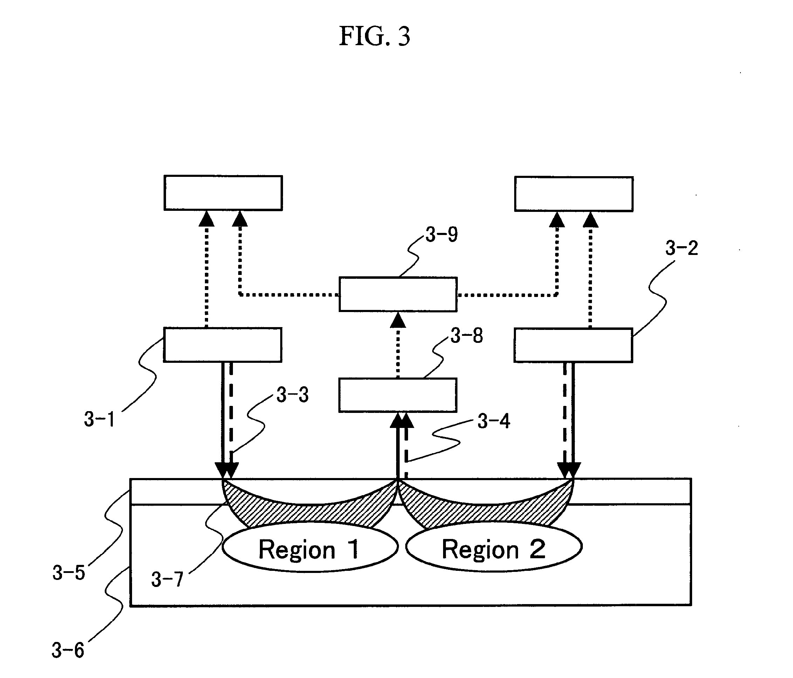

Next, a methodology which measures and images a blood amount variation accompanying brain activity will be described. (FIG. 3)

FIG. 3 is a diagram showing two illumination optical fibers on either side of one detection optical fiber in the arrangement of illumination optical fibers and detection optical fibers shown in FIG. 2. 3-1 and 3-2 are light sources, whereof the intensity is modulated at respectively different frequencies. 3-3 are illumination optical fibers and 3-4 are detection optical fibers, these being arranged at intervals of 30 mm. 3-5 and 3-6 schematically show brain structures, i.e., the skull and cerebral cortex, respectively. The banana shaped-region indicated by 3-7 shows the path of the detection light which disperses through each illumination optical fiber/detection fiber pair. Since a living body tissue (in particular, the skull) strongly disperses light, the light illuminated by the illumination optical fibers loses directivity from the illumination position, and is scattered in all directions. As shown in this diagram, light which has been propagated between the light illumination position and light detection position which are 30 mm apart, spreads out in a banana shape, and can be detected as reflected light. The detected light is converted from an optical signal into an electric signal by the detector indicated by 3-8 (comprising a photodiode or photomultiplier). The converted electric signal is processed by a lock-in amplifier indicated by 3-9.

In this method, the reflected light intensities of the light illuminated from two light sources are detected simultaneously, and can be distinguished separately. Here, if the concentration of oxygenated Hb or deoxygenated Hb varies in Region 1 or Region 2 in connection with brain activity, the reflected light intensity will vary. The blood amount variation in Region 1 and Region 2 can then be computed from this reflected light intensity. In FIG. 3, there are two pairs of illumination optical fibers and detection optical fibers, but the case will be described for one pair thereof. Let the optical intensity reaching the detection optical fibers at each time be I(t). In this living body light measurement method, to measure the concentration change of a living body metabolite, postulate the existence of two states, State 0 and State 1, and let the optical intensity in each state by 10 and I1. Here, let State 0 be a state representing a base condition wherein the living body is at rest, and let State 1 be a state where a motor area is active due to finger movement, for example. A variation ($\Delta A$ (t)) of the absorbance inside the living body at different times with respect to light sources of two wavelengths used for measurement satisfies the following relation.

$$\text{Equation 1: } \Delta A(t) = -\ln(I_1(t)/I_0(t)) = \varepsilon_{oxy}\Delta C_{oxy}(t)L + \varepsilon_{deoxy}\Delta C_{deoxy}(t)L$$

Here, L in Equation 1 is the average optical path length between the light source and the detector.
$\varepsilon$oxy and $\varepsilon$deoxy in this formula show the molecular extinction coefficient of oxygenated Hb and deoxygenated Hb respectively. The concentration change of oxygenated Hb or deoxygenated Hb (respectively $\Delta C$oxy, $\Delta C$deoxy), accompanying brain activity may be found by applying this Equation 1 to each wavelength.

$$\text{Equation 2: } \begin{pmatrix} \Delta C_{oxy}(t) \\ \Delta C_{deoxy}(t) \end{pmatrix} = \begin{pmatrix} \varepsilon_{oxy}^{\lambda 1} & \varepsilon_{deoxy}^{\lambda 1} \\ \varepsilon_{oxy}^{\lambda 2} & \varepsilon_{deoxy}^{\lambda 2} \end{pmatrix}^{-1} \begin{pmatrix} \dfrac{-\ln(I_1^{\lambda 1}(t)/I_0^{\lambda 1}}{L^{\lambda 1}} \\ \dfrac{-\ln(I_1^{\lambda 2}(t)/I_0^{\lambda 2}}{L^{\lambda 2}} \end{pmatrix}$$

In practice, it is difficult to determine L, so writing:

$$\text{Equation 3: } \begin{pmatrix} \Delta C_{oxy}^n \\ \Delta C_{deoxy}^n \end{pmatrix} = L \begin{pmatrix} \Delta C_{oxy} \\ \Delta C_{deoxy} \end{pmatrix}$$

for C' which is a unit which having the dimensions of concentration multiplied by distance,

$$\text{Equation 4: } \begin{pmatrix} \Delta C'_{oxy}(t) \\ \Delta C'_{deoxy}(t) \end{pmatrix} = \begin{pmatrix} \varepsilon_{oxy\lambda 1} & \varepsilon_{deoxy\lambda 1} \\ \varepsilon_{oxy\lambda 2} & \varepsilon_{deoxy\lambda 2} \end{pmatrix}^{-1} \begin{pmatrix} -\ln(I_{1\lambda_1}(t)/I_{0\lambda_1}) \\ -\ln(I_{1\lambda 2}(t)/I_{0\lambda_2}) \end{pmatrix}$$

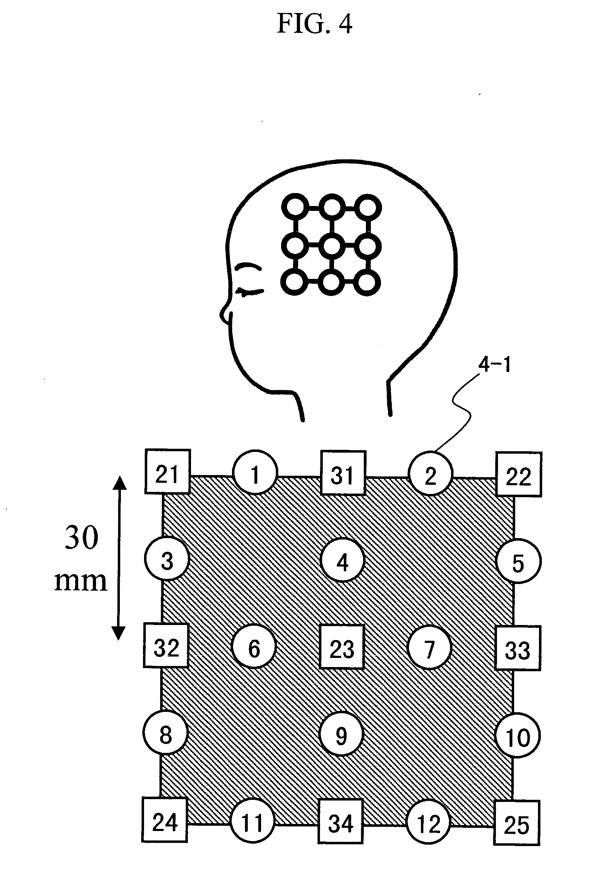

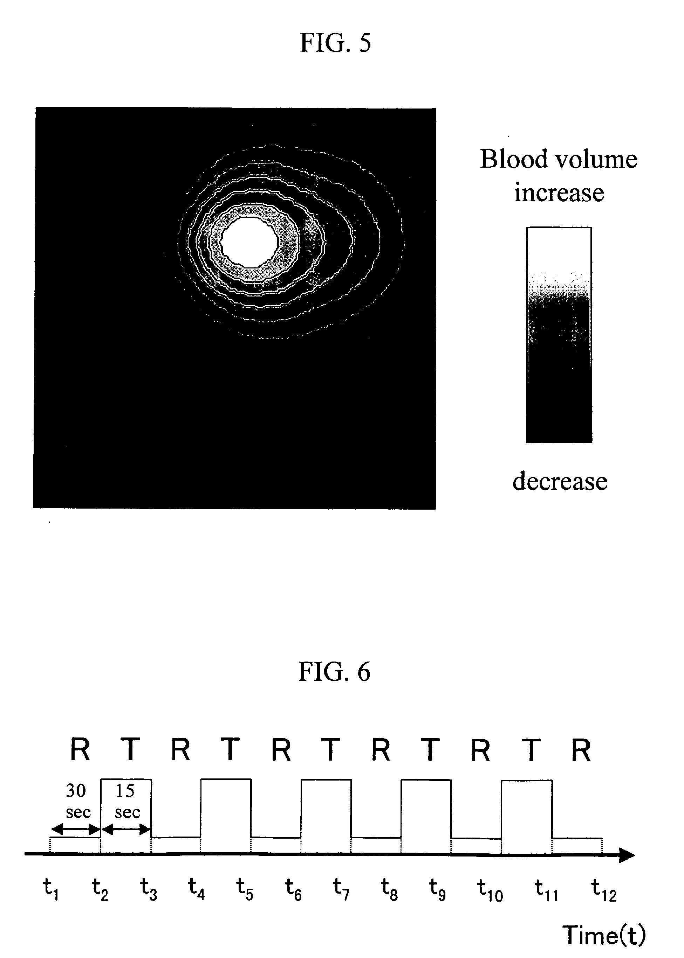

may be computed.
Next, the measurement method using this FIG. 3 and the method of preparing a topographic image showing the distribution of the concentration change of oxygenated Hb and deoxygenated Hb computed using Equation 4, will be described (FIG. 4). In this FIG. 4, the case is shown where five illumination optical fibers are disposed at positions 21, 22, 23, 24 and 25 in the figure, and four detection optical fibers are likewise disposed at positions 31, 32, 33 and 34 in the figure. In such an arrangement, there are many pairs of illumination optical fibers and detection optical fibers, there being a total of 12 pairs of illumination optical fibers and detection optical fibers at intervals of 30 mm. The midpoints of these optical fiber pairs are defined as sampling points which give positional information regarding the concentration change of oxygenated Hb and deoxygenated Hb detected by one optical fiber pair. Under this assumption, in FIG. 4, there are a total of 12 sampling points (4-1). By performing spatial interpolation processing on the concentration change of oxygenated Hb and deoxygenated Hb at these 12 points, the topographic image shown in FIG. 5 is obtained. This image is a topographic map which displays physical quantities having other dimensions on plane coordinates. In the measurement system shown in FIG. 3, data can be sampled about every 100 milliseconds, and this can also be represented as a moving image at this sampling interval.

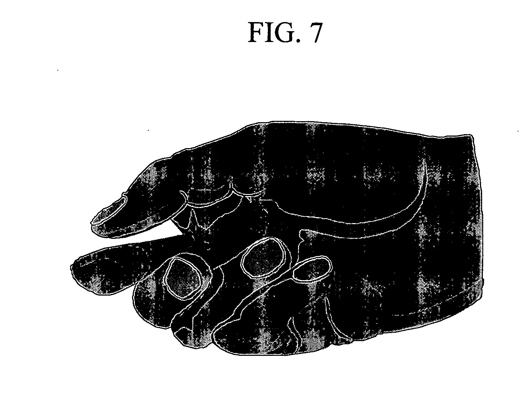

Next, the activation method (paradigm) used to activate the brain will be described. In this living body light measurement system, in order to induce a metabolite concentration change in the living body, a subject is given a task. An example of this paradigm and a task sequence is shown in FIG. 6. In the figure, T and R are respectively a task period (period wherein the subject performs a task), and a rest period (state wherein the brain is in a state such as keeping still, which is different from the task period). For example, describing this using the human hand shown in FIG. 7, in the task period, the subject performs a task wherein the thumb and fingers other than the thumb such as the index finger, middle finger, third finger and little finger are randomly brought in contact at a speed of 1 Hz. On the other hand, in the rest period, unlike the task period mentioned above, rest is maintained without the fingers in contact. The difference between the task period and rest period is only whether or not finger movement is performed, so the finger movement area of the temporal lobe is activated, and the concentration of oxygenated Hb in this active part varies, as described in Non-patent document 3. In actual measurements, T and R are set to various values depending on the test purpose or content, but in general, T = approx. 15 seconds, R = approx. 30 seconds. To reduce the noise of the measured signal, the task is performed repeatedly and synchronous addition processing is performed on the measured reflected light intensity change. The repeat number of T used in an actual clinical setting is from 5 to 10 times, and the time required for each measurement is about 10 minutes.

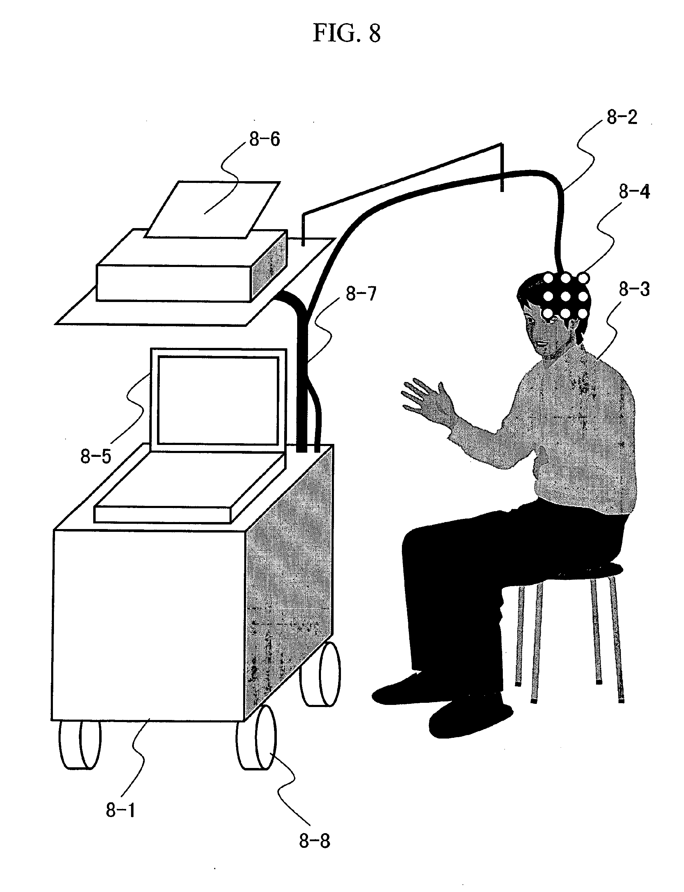

FIG. 8 shows a measurement scene using an actual measurement system. The light source array and detector array shown in FIG. 2 are stored in a case (8-1), and a fiber array (8-2) which includes the illumination optical fibers and detection optical fibers is connected to a helmet (8-4) on the head of the subject (8-3). Measurement results can be promptly perused by using a printer (8-6) connected to the computer (8-5) which prints the measurement results. The optical fiber array described above is connected to a brace (8-7) supporting this printer, and since the case has casters (8-8), it is highly portable. Hence, the spatial relationship between the subject and this measurement system can be set freely, and an examination can be performed not only in an outpatient examination room of a medical institution, but also in an operating room or sickroom.

[Patent document 1] JP-A No. 103434/1996
[Patent document 2] JP-A No. 98972/1997
[Non-patent document 1] E. Watanabe, A. Maki, F. Kawaguchi, Y. Yamashita, H. Koizumi, Y. Mayanagi, " Noninvasive Cerebral Blood Volume Measurement During Seizures Using Multichannel Near Infrared Spectroscopic Topography", Journal of Biomedical Optics, 2000, July, and 5(3) P.287-290.
[Non-patent document 2] E. Watanabe, A.Maki, F.Kawaguchi, K. Takashiro, Y.Yamashita, H. Koizumi and Y. Mayanagi, "Non-invasive assessment of language dominance with Near-Infrared Spectroscopic Mapping", Neurosci. Lett.256 (1998).
[Non-patent document 3] Atsushi Maki, Yuichi Yamashita, Yoshitoshi Ito, Eijyu Watanabe, Yoshiaki Mayanagi and Hideaki Koizumi, "Spatial and temporal analysis of human motor activity", Medical Physics, Vol.22 (No.12), pp. 1997-2005(1995).

SUMMARY OF THE INVENTION

In this living body light measurement method, to reduce noise in the measurement result, the same task (problem) can be repeated plural times, and the subject may be considered to be in either of the following two states under test.

In the first state, the subject performs a task specified by the doctor or examining technician during a task period, and rests during a rest period as directed. In this case, if the doctor and examining technician analyze measurement result data after measurements have been completed, it is possible to evaluate the brain activity of the subject. On the other hand, the subject may perform actions during the task period or rest period which are different from those specified. For example, since the contact state of the helmet on the head will change if the subject coughs during measurement, a noise (artifact) will be detected. Also, if the task is simple, the subject may become bored, and although a right hand movement has been specified as a task, the subject may perform a different task (for example, move the left hand), and this may not be detected as a noise (artifact). If it is not detected as an artifact, and the subject performs a different task from that specified by the doctor or examining technician, a different area from the brain functional part which was being studied is activated, and the correct test result may not be obtained.

Thus, if data for a period when noise has been detected, or a period where the correct task is not performed although noise was not detected, is used for data analysis, the correct data cannot be obtained. In order to obtain correct data, the time when noise occurred in this way needs to be specified and it must be removed from the object of the synchronous addition, but an effective method of doing this had not yet been established.

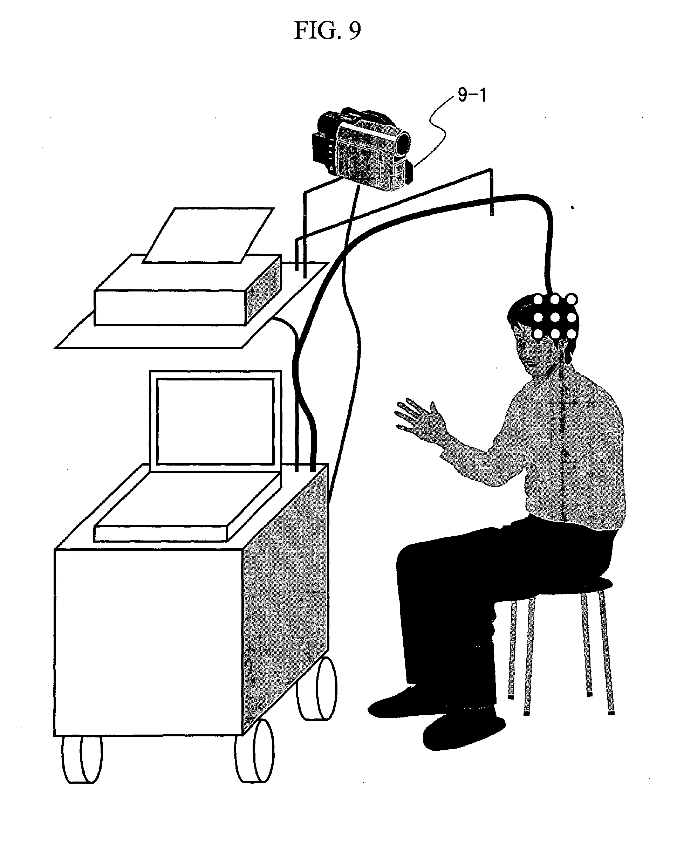

On the other hand, the test subject might be videotaped. A VTR camera can be set up, and applied in the aspect shown in FIG. 9. Unlike the case of an MRI device or brain magnet meter which require special examination rooms for measurement, in this living body light measurement method, there is no need to be concerned about electromagnetic noise. Hence, a VTR camera (9-1) can be arranged near the measurement system, and this VTR camera can be installed in any position relative to the subject. For this reason, taking the case of the measurement of finger motor function which was described using FIG. 9 as an example, the movement of the finger alone can be photographed, or the whole subject can be photographed.

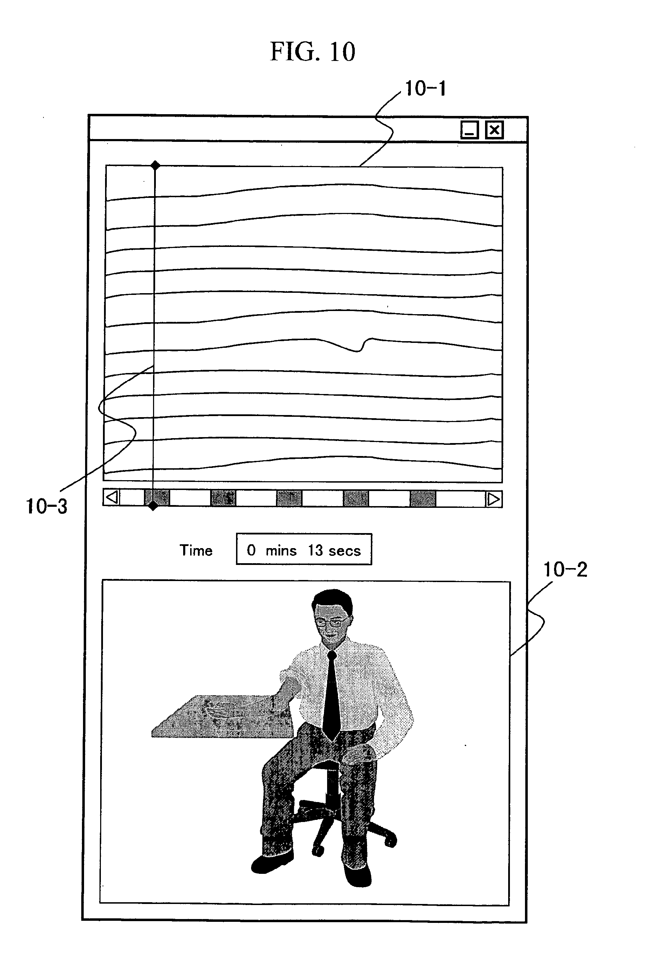

10-1 in FIG. 10 is a time course showing a measurement result, and in this figure, a reflected light intensity change for each wavelength at each sampling point is shown. 10-2 is an image showing the subject's behavior.

The line (10-3) in 10-1 mentioned above specifies the correspondence between the image reproduction time and the time on the time course, so problems occurring on the time course can be identified by reproducing the image and time course in synchronism.

However, even if the videotaped result and measurement result are reproduced in synchronism by the method shown in FIG. 10, this may not be useful for the doctor or examining technician. This is because, although the topographic image shown in FIG. 7 can be obtained in several minutes after measurements have been completed, the image shown in 10-2 needs to be reproduced during the whole of the 10 minutes or so from measurement start to finish, so the measurement result cannot be verified promptly after measurements have been completed. Hence, the image results between each task cannot be directly compared, and an efficient performance check cannot be made.

Methods were therefore considered of resolving these problems, i.e., shortening the reproduction time to check measurement images, allowing an efficient performance check to be made, and deleting data for periods containing noise or periods where tasks were not correctly performed in a short time.

To solve the above problems, the present invention provides a living body light measurement system, wherein the behavior of the subject is photographed in a first trial period (rest period wherein the subject is at rest and task period wherein a task is given to the subject) and a second trial period (rest period wherein the subject is a rest and task period wherein a task is given to the subject), and a moving image in the first trial period and a moving image in the second trial period are displayed effectively simultaneously. Consequently, a performance check can be made in the time required for one trial period, and image data or measurement data between tasks can be directly compared.

According to the present invention, the measurement state and measurement results of the subject in each trial period are verified simultaneously, so a performance check in each trial period can be performed efficiently, and the moving image reproduction time can be shortened. Further, data for trial periods containing noise or trial periods wherein a task is not correctly performed can be eliminated in a shorter time than in the prior art.

BRIEF DESCRIPTION OF THE DRAWINGS

FIG. 1 shows an image (1) of the living body light measurement system provided by the present invention.

FIG. 2 shows the construction of the living body light measurement system.

FIG. 3 shows a method of measuring blood volume variation due to brain activity.

FIG. 4 shows a method of preparing a topographic image showing a concentration variation distribution of oxygenated Hb and the oxygenated Hb.

FIG. 5 is a topographic image.

FIG. 6 is a task sequence during brain function measurement.

FIG. 7 is a human hand.

FIG. 8 is a scene during brain activity measurement using the living body light measurement system.

FIG. 9 shows the construction of a living body light measurement system provided with a VTR camera.

FIG. 10 shows a method of displaying results obtained by VTR photography and measurement results for a subject according to the prior art technique.

FIG. 11 shows the method of performing measurements and the method of processing measurement results.

FIG. 12 is an image (2) of the living body light measurement system provided by the present invention.

FIG. 13 is an image (3) of the living body light measurement system provided by the present invention.

FIG. 14 is an image (4) of the living body light measurement system provided by the present invention.

FIG. 15 is a diagram showing the construction of a measurement system, comprising a living body light measurement system and a different living body light measurement system from this living body light measurement system, and having a function to photograph the subject.

FIG. 16 shows the construction of a finger movement function measurement system.

FIG. 17 shows the method of fitting the finger movement function measurement system to the living body.
FIG. 18 is a finger movement waveform (1).
FIG. 19 is a finger movement waveform (2).
FIG. 20 shows time variations of T1 and T2.
FIG. 21 is an image of the measurement system shown in FIG. 15.
FIG. 22 is an image data storage folder.

## DETAILED DESCRIPTION OF THE PREFERRED EMBODIMENTS

First Embodiment

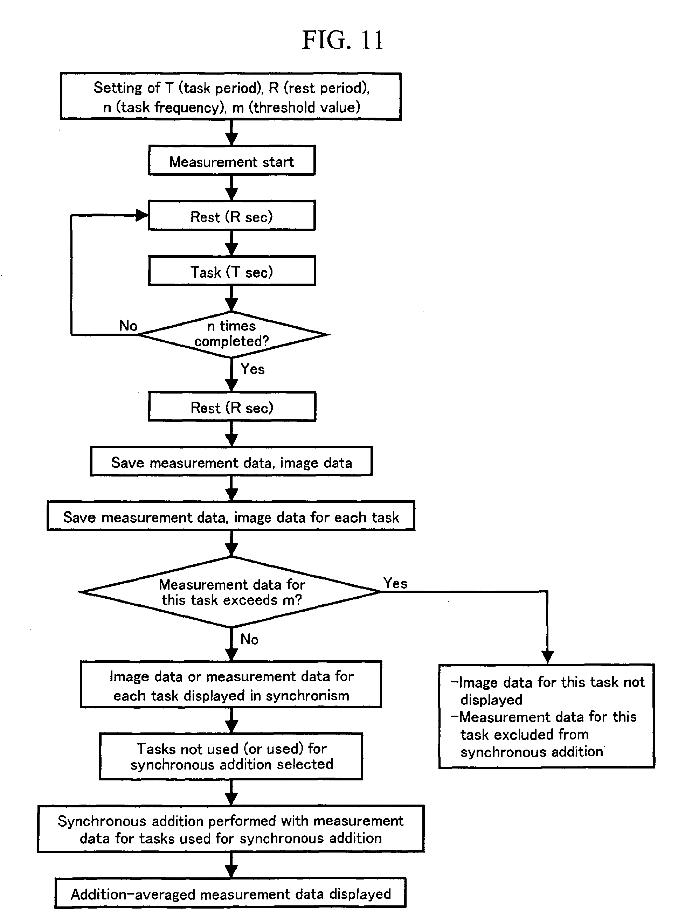

One aspect for implementing the present invention will now be described referring to the first embodiment. Firstly, the measurement method and measurement result processing method shall be described using the flowchart shown in FIG. 11. Firstly, if the sequence shown in FIG. 6 is used, a task time (T), rest time (R) and task repeat frequency (n) are set beforehand. According to this aspect, in FIG. 6, n = 5. Based on the set frequency and time, rests and tasks are alternately repeated n times. After measurements are complete, firstly, the measurement data is stored. These measurement data are the time variation characteristics of the reflected light intensity for each wavelength at each sampling point. After these measurement data have been stored, image data photographed during the measurement period are also stored. Here, both the measurement data and image data are time sequence data, but in order to maintain data synchronism, the measurement start time, task start time and end times are recorded in both types of data as markers when the data are acquired. Next, the image data is divided up into measurement sequences, and stored. For this purpose, five image files containing each time are stored in a folder.

$$Equation\ 5:\ t1 \leq t \leq t4$$

$$t3 \leq t \leq t6$$

$$t5 \leq t \leq t8$$

$$t7 \leq t \leq t10$$

$$t9 \leq t \leq t12$$

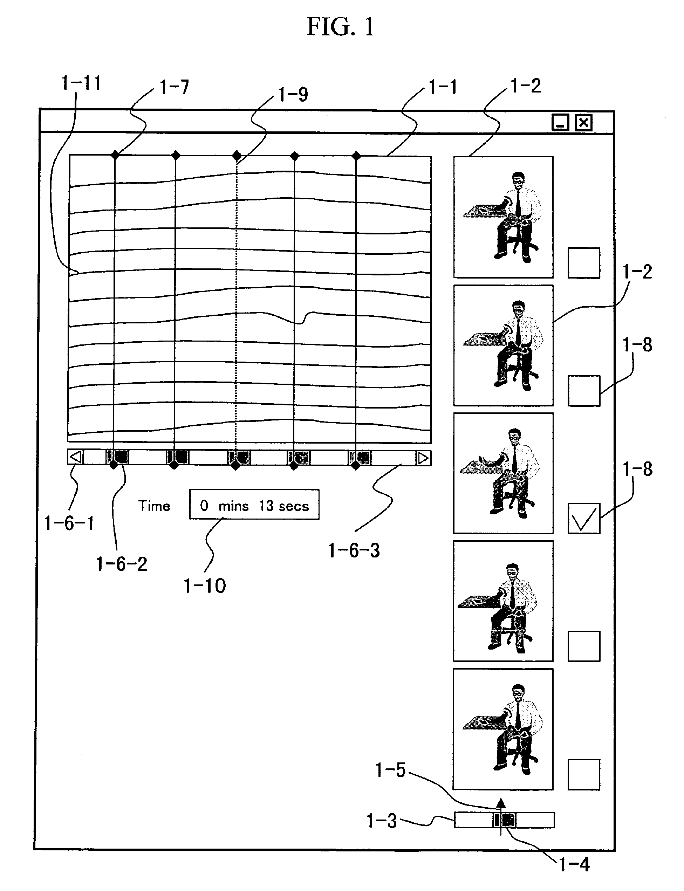

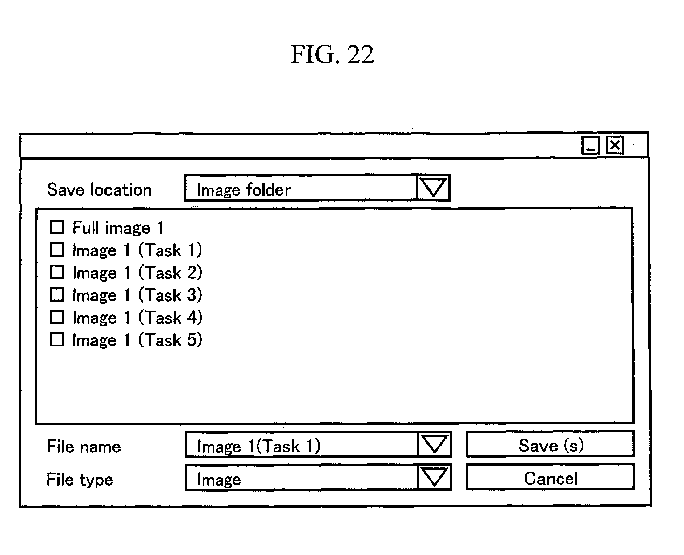

FIG. 22 shows the stored data. In this folder, in addition to the photographed image file "Full Image 1", "Image 1 (Task 1)", "Image 2 (Task 2)", "Image 3 (Task 3)", "Image 4 (Task 4)", "Image 5 (Task 5)" are also stored. In the flowchart shown in FIG. 11, these files are used for the image shown in FIG. 1 after "synchronous reproduction of image data or measurement data for each task". In the image shown in FIG. 1, the time courses of the measurement data shown by 1-1 and the image shown by 1-2 are displayed alongside each other. In this aspect, five images are arranged in the order first task, second task.... fifth task. The bars shown by 1-3, 1-4 respectively indicate rest periods and task periods. The arrow shown by 1-5 indicates the time for each image shown by 1-2, and each image is reproduced effectively simultaneously (here, effectively means that synchronization errors due to mechanical noise or mechanical performance limits, and images which do not exceed the users visual perception of synchronism, are allowed). In other words, the five images are arranged in order of task start time (describing this using the measurement sequence shown in FIG. 6, for these five images, t2 for the first, t4 for the second, t6 for the third, t8 for the fourth and t10 for the fifth), and are reproduced simultaneously.
As the time course shown by 1-1 is time sequence data, as shown in FIG. 6, rest periods and task periods are set alternately, and displayed by the task period and rest period display bar shown by 1-6-1. The line shown by 1-7 displaces in synchronism with the arrow shown by 1-5. In this way, since the lines showing the relation between the images and times at which the images are displayed displace in synchronism on the images and time course, the relation between the data indicated by the time course and the images can be clearly appreciated by the user. 1-8 is a check box, and in this aspect of the invention, a check mark is placed in this box. This indicates that, in this aspect, in the third task, the subject's behavior was very different from the first, second, fourth and fifth tasks, so it was selected by the user to be excluded from synchronous addition when data processing was performed.
When this check box is ticked, the line which shows synchronism of the time shown by 1-9 becomes a different type of line from the other lines, so the user can be informed of non-validity of data on the time course. In the display method shown in FIG. 1, when T = 15 seconds, R = 30 seconds and the task repeat frequency is 5 (sequence shown in FIG. 6), a display time of 255 seconds could be reduced to 75 seconds (30+15+30). The lines shown by 1-11 are the time sequence variation (time course) of the detection light intensity obtained by converting the detection light described in FIG. 3 to an electrical signal, each line corresponding to measurement data at the sampling points (4-1) shown in FIG. 4 (in this aspect, there are 12 lines (1-11) corresponding to 12 sampling points (4-1)). Also, 1-10 shows how many seconds the lines shown by 1-7 and 1-9 are after the task start, or shows this time, and this changes together with the display times in synchronism with the positional variation of the lines shown by 1-7 and 1-9.

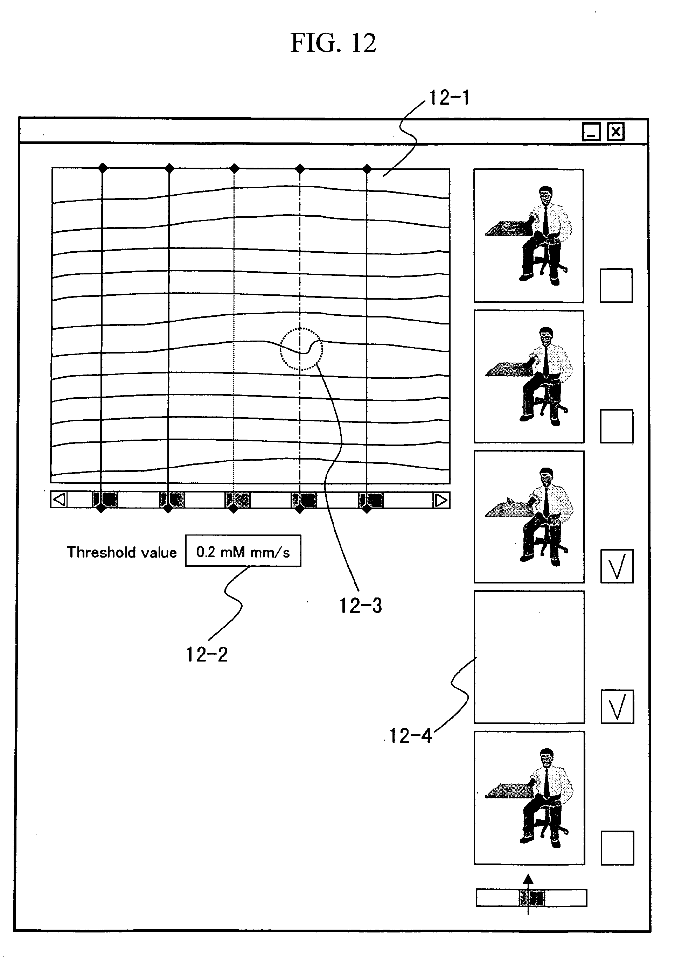

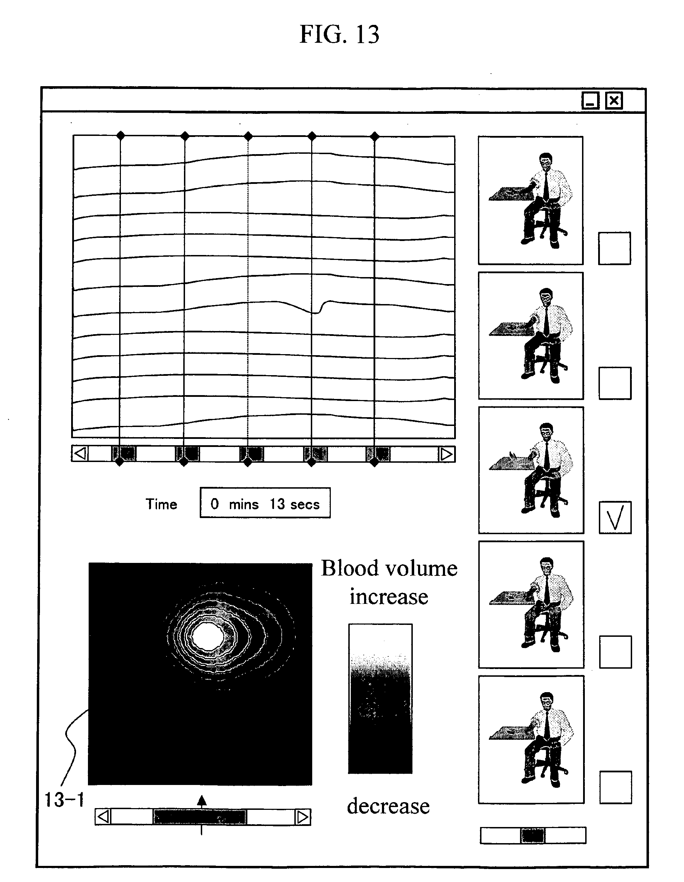

Next, a modification of FIG. 1 will be described referring to FIG. 12 and FIG. 13. In FIG. 12, the presence of noise in the data displayed by the time course (12-1) is automatically determined, and tasks containing this noise are not displayed as images or the corresponding task measurement data is excluded from the synchronous addition. As an example of the determination algorithm when this noise is present, assume it is determined that noise is present when the blood volume variation per unit time exceeds a preset threshold value (m). 12-2 is a threshold value setting part which inputs this threshold value. As an example, if "0.2 mM mm/0.1 sec" is input, it is considered that noise is present when the blood volume has changed by $\pm 0.2$ mM mm in 0.1 seconds. The computation of this blood volume variation uses the calculation algorithms shown by Equations (1) to (4). In this aspect, noise appears at the sampling point shown by 12-3, and the measurement for the fourth task containing this time is invalidated. Therefore, at the position shown by 12-4, an image is not displayed. Also, the measurement data for the fourth task can be excluded from the synchronous addition.

FIG. 13 shows a modification of FIG. 1 and FIG. 12. The image is obtained by displaying the images in each task period and the topographic image (13-1) acquired by synchronous addition processing of the measurement data in each task period alongside each other, and reproducing them effectively in synchronism. In this aspect, a task (one of the five tasks) to be excluded from the synchronous addition may be selected while viewing the images, and the topographic images prepared from the selection results viewed immediately. Using the method shown in this aspect, for example, the different topographic moving images obtained when the third task is included in or excluded from the addition can easily be compared by the user, and the results of this performance check can therefore be more easily reflected in the data processing method. A check mark may also be placed to include a task in the synchronous addition. Here, in the drawing shown in this aspect, a check mark is placed against the third task. Hence, by synchronously adding the transmitted light intensities of each channel in the periods $t1 \le t4$, $t3 \le t6$, $t7 \le t10$, $t9 \le t12$ shown in Equation 5, computing $\Delta C'$ using Equations 1-4 and performing a spatial interpolation using positional information for each channel, a topographic image can be displayed as shown in 13-1 of this figure.

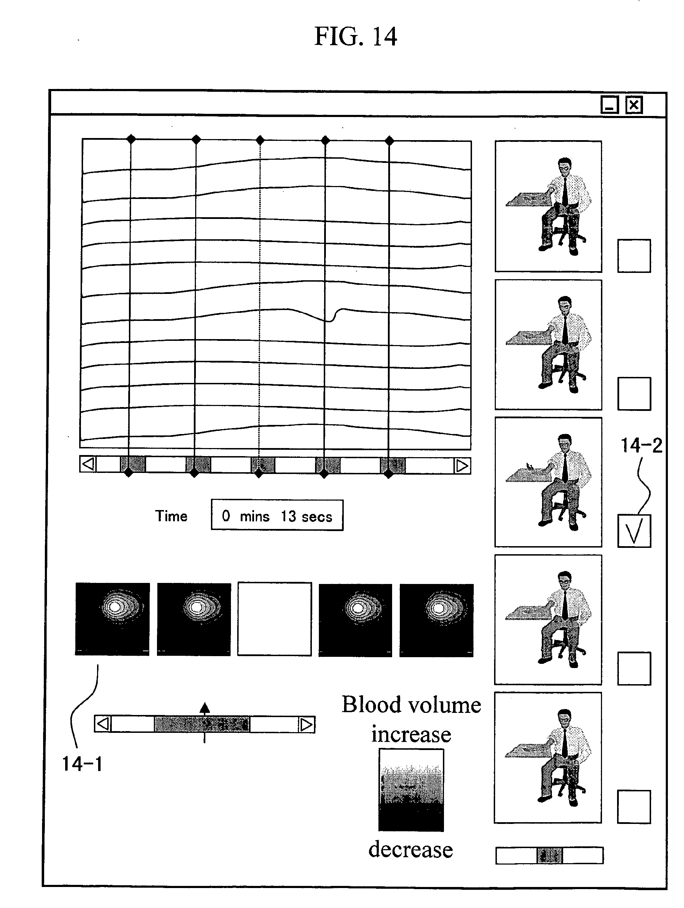

FIG. 14 is a modification of FIG. 13, showing topographic images (14-1) for each task period which was repeated alongside each other. In this figure, as shown in 14-2, a check is placed in the check box for the third task. Therefore, the third topographic image is not displayed. As a result, by showing only the first, second, fourth and fifth topographic images for measurement processing, the number of images which must be verified by the user can be reduced.

The aforesaid image display and data processing may also be performed by installing a program to execute these functions in a prior art living body light measurement system not having these functions.


Second Embodiment


As the living body light measurement system uses light, there is no interference from other electromagnetic energies. Consequently, simultaneous measurements can be performed in measuring devices which use electromagnetic energy other than light. An example of such an aspect is described below.

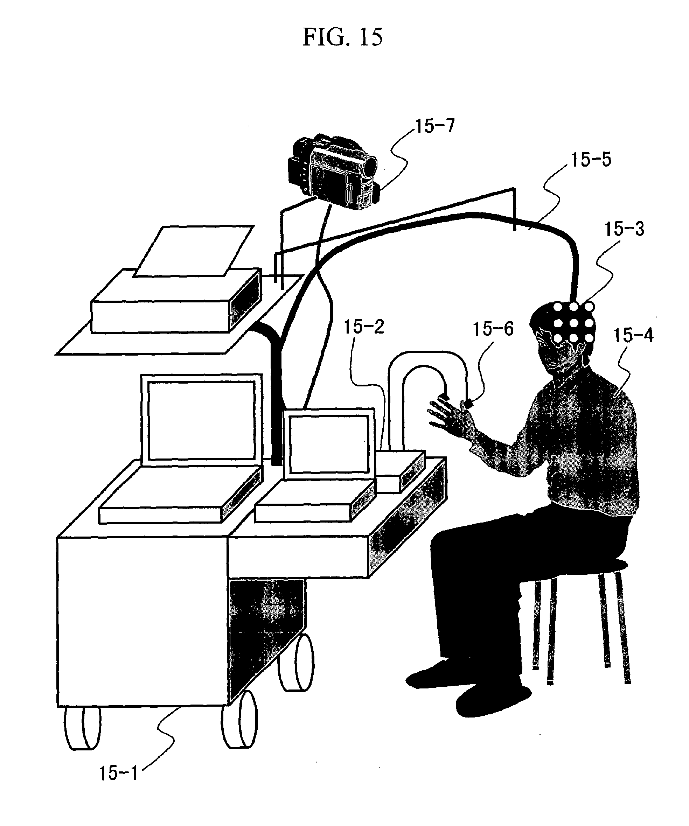

FIG. 15 shows a system wherein the living body light measurement system shown in FIG. 9 is installed together with a living body measurement system different from this living body measurement system. In this aspect, the case is described where a different finger movement function measurement system (15-2) is installed alongside the living body light measurement system (15-1), but it should be understood that the invention is not limited to this aspect. The subject (15-4) fitted with the helmet (15-3) is connected to the living body light measurement system by an optical fiber array (15-5). The finger of the subject is connected to a sensor (15-6), this sensor and the finger movement function measurement system being connected together. The living body light measurement system has a video camera (15-7) for photographing the subject's behavior.

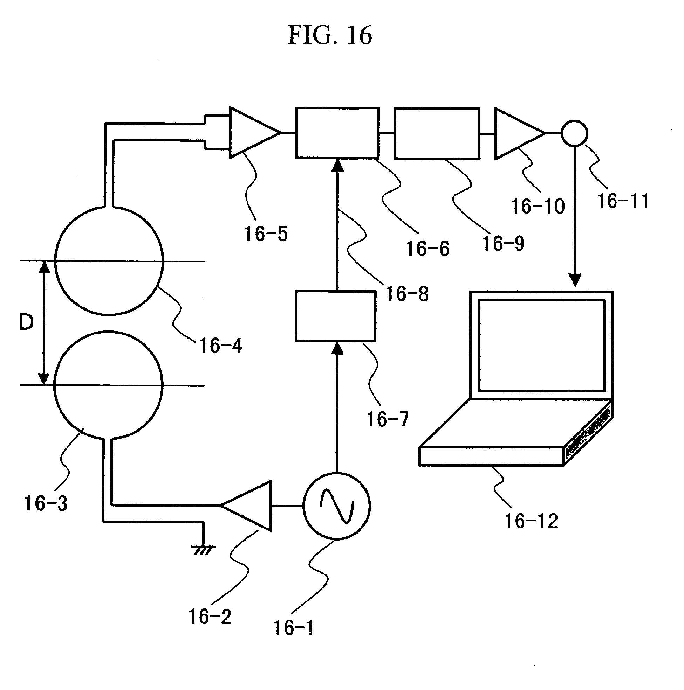

Next, the construction of this finger movement function measurement system will be described referring to FIG. 16. An AC voltage having a predetermined frequency (e.g., 20 kHz) is produced by an AC generation circuit (16-1). The generated AC voltage having a predetermined frequency is converted to an alternating current having a predetermined frequency by an AC generation amplifier circuit (16-2). The alternating current flows through a transmitting coil 2 fitted to the living body. A magnetic field generated by a transmitting coil (16-3) generates an induced electromotive force in a receiver coil (16-4) fitted to the living body. The generated induced electromotive force (having the same frequency as that of the alternating current having a predetermined frequency generated by the AC generation circuit (16-1)) is amplified by a preamplifier circuit (16-5). After amplification, the signal is input to a detection circuit (16-6). In this detection circuit, detection is performed at the predetermined frequency or twice the frequency generated by the AC generation circuit, so after the phase has been adjusted by a phase adjustment circuit (16-7), the output of the AC generation circuit is input to a reference signal input terminal of the detection circuit 4 as a reference signal (16-8).

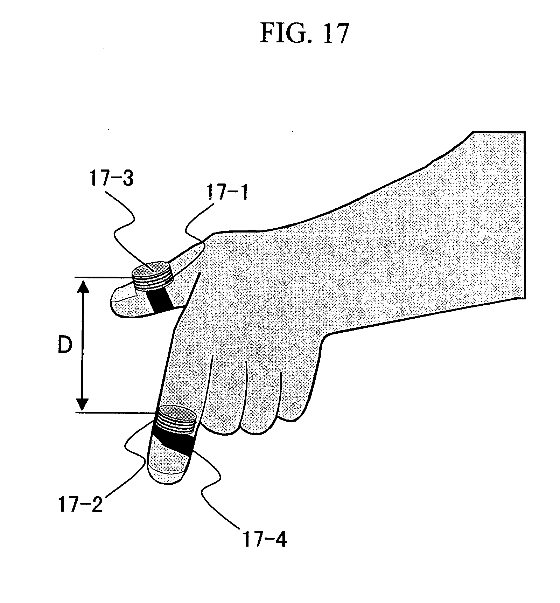

If detection is performed at twice the predetermined frequency, the phase adjustment circuit (16-7) is not absolutely necessary. A simple circuit which detects at twice the frequency may be obtained by setting the predetermined frequency of the AC generation circuit (16-1) to twice the frequency, converting to half the frequency by frequency division, inputting this to the AC generation amplifier circuit (16-2), and inputting a signal having twice the frequency of the predetermined frequency of the AC generation circuit (16-2) to the reference signal input terminal of the detection circuit (16-6) as a reference signal (16-8). The output of the detection circuit (16-6) is passed through a low pass filter circuit (16-9) and amplified by an amplifier circuit (16-10) to obtain a desired voltage to give an output (16-11). The output (16-11) is converted to digital data by an analog/digital converter board (AD board) built into a computer (16-12), and input to this computer. In the aforesaid example, a voltage corresponding to a relative distance D between the receiver coil (16-4) and transmitting coil (16-3) fitted to the living body is detected. The method of fitting this finger movement function measurement system to the living body will now be described referring to FIG. 17.

The transmitting coil (17-1) is fitted to the upper part of the thumb, and the receiver coil (17-2) is fitted to the upper part of the index finger. The transmitting coil is wound on a coil attach component (17-3), and is connected to the AC generation amplifier (16-2) shown in FIG. 16. The attach components are attached to bands (17-4), elastic bands or sponges being used to absorb individual differences in the size (thickness) of the fingers. In the construction shown in this diagram, an output corresponding to the relative distance D between the thumb and index finger can be obtained. The fingers to which the receiver coil and transmitting coil are fitted are not limited to the thumb and index finger, and may be any desired fingers. Further, the transmitting coil and receiver coil may be fitted to the upper lip and lower lip so that mouth movements can be detected.

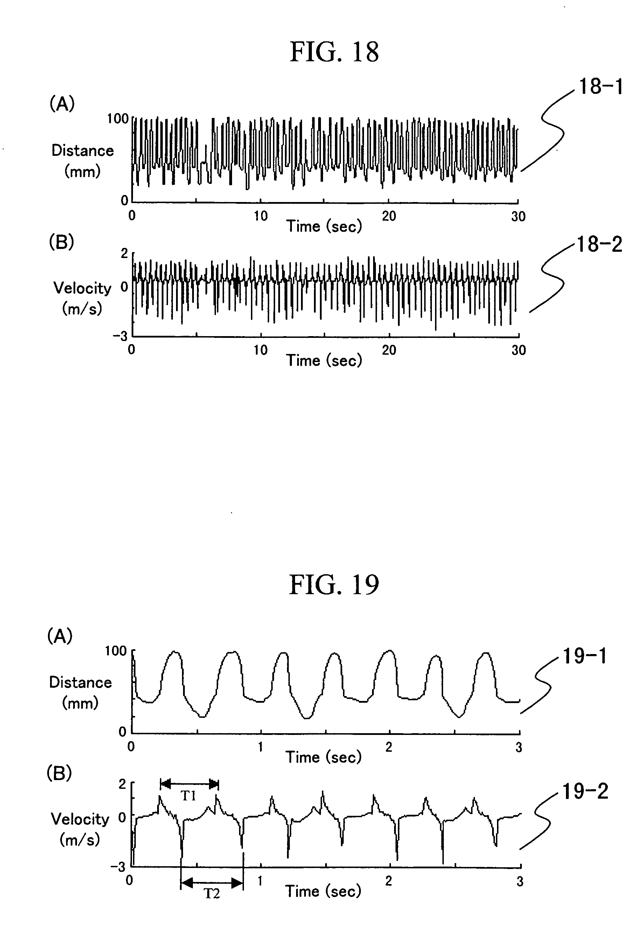

FIG. 18 shows the waveform actually obtained when these springs were fitted to the thumb and index finger, and a person suffering from Parkinson's disease was directed to perform finger tapping of the thumb and index finger (where the thumb and index finger are repeatedly separated and brought together) with as rapid and wide finger separation as possible. The relation between the potential difference obtained from the distance between the two coils, and the actual distance between the thumb and index finger, was obtained beforehand and used to correct the waveform shown in FIG. 18. In FIG. 18, the waveform shown by 18-1 is data converted to the relative distance D and its velocity waveform (first differential waveform (dX/dt) in the time direction of a distance X). In FIG. 19 which is an enlargement of this figure from 0 seconds to 3 seconds, 19-1 and 19-2 likewise show measurement data and velocity waveforms. T1 and T2 shown in 19-2 are respectively a time width indicating a detected time difference between adjacent maximum peaks in the velocity waveform, and a time width indicating a detected time difference between adjacent minimum peaks in the velocity waveform.

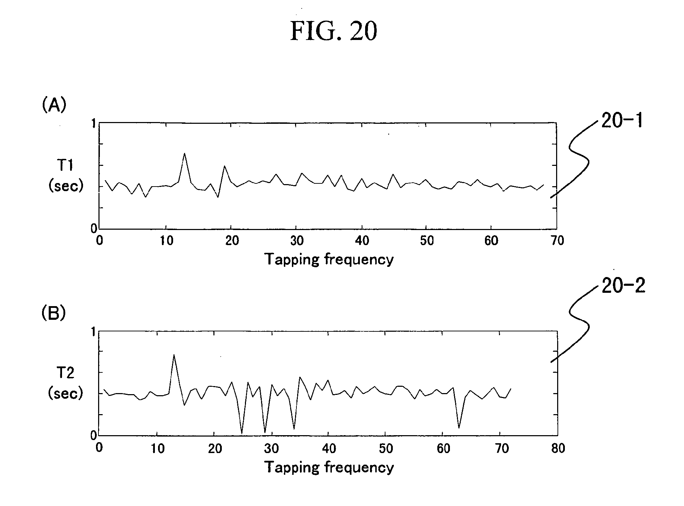

T1 and T2 show plots for each tapping frequency (FIG. 20).

20-1 and 20-2 are plots of T1, T2 for each tapping frequency. From this figure, it is seen that T1 has a tapping frequency centered on 0.4 seconds. On the other hand, it is seen that T2 also fluctuates effectively around 0.4 seconds as in the case of T1, but there are disturbances at several points. As shown in FIG. 18, by plotting the time variation of tapping, temporal disturbances of tapping can be visually appreciated.

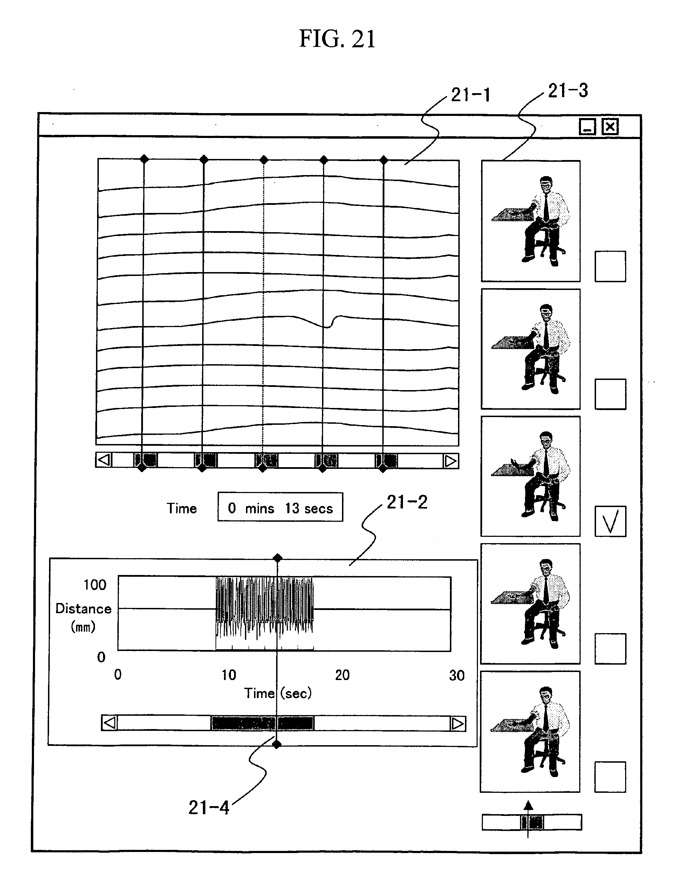

In the measurement system shown in FIG. 15, a video camera is provided, so measurement results obtained with the living body measurement system, results obtained with a living body measurement system other than the living body measurement system which allows simultaneous measurements shown in FIG. 15 and photographic results can be displayed and reproduced simultaneously, so the measurement results obtained by the living body measurement devices and subject performance checks can be performed simultaneously. FIG. 21 shows the display method. This figure has a time course (21-1) showing the measurement results obtained using light, a finger movement waveform (21-2) obtained from the finger movement function measurement system shown in FIG. 15, and photographs (21-3) of the subject. In the lower part of this image, to indicate the time, a line (21-4) showing the relation between task periods, rest periods and the present time is marked. This line changes its position with the elapsed time, and the five images are also reproduced effectively simultaneously. In this figure, the time course (21-1) showing measurement results obtained using light, the finger movement waveform (21-2) obtained from the finger movement function measurement system shown in FIG. 15 and the photographs (21-3) of the subject are all displayed, but only one or two thereof may be displayed.

As a result, a measurement system is obtained which allows living body functions to be measured simultaneously by plural modalities, which compares subject behavior for each repetitive task, and which permits the doctor or an examining technician to obtain a diagnosis more easily. In other words, according to this aspect, in addition to photographs of the subject, data reliability can be enhanced by directly measuring, for example, subject movement, motion, actions and phase change information.

This living body light measurement method moreover allows simultaneous measurement with other living body measurement techniques (brainwaves, functional magnetic resonance imaging devices, positive electron dislocation picturizing methods), and has the advantage that it can be implemented even in a living body light measurement technique which also employs a VTR. Consequently, the scope of physiological information which can be acquired is increased.

In the living body light measurement system which measures phenomena such as in-vitro metabolite concentration variations, e.g., blood volume variations due to brain activity, the results of computing these concentration variations with subject behavior can be compared easily and in a short time.

**Claims**

1. A living body measurement system by light, comprising:

   a light illumination device which illuminates a subject
   a light detector which detects light emitted from said light illumination device which has dispersed inside said subject,
   a calculation part which calculates a metabolite concentration change of said subject from a measurement result detected by said light detector, and
   an imaging device which captures a moving image of said subject under measurement,

   wherein said metabolite concentration change in a first trial period and second trial period is measured, said first trial period and said second trial period having a rest period where said subject is left at rest, and a task period where a task is given to said subject, and
   said device further comprises a display which displays the moving image in said first trial period and said second trial period effectively in synchronism.

2. The living body light measurement system according to Claim 1, wherein said device displays said metabolite concentration change together with said moving image effectively in synchronism.

3. The living body light measurement system according to Claim 2, wherein said display displays said metabolite concentration change as a topographic image.

4. The living body light measurement system according to Claim 2, having a setting part which sets whether said moving image is displayed or is not displayed in each trial period.

5. The living body light measurement system according to Claim 4, wherein metabolite concentration changes are addition-averaged over the trial periods wherein display is selected by said setting part, and the addition average of said metabolite concentration changes is displayed.

6. The living body light measurement system according to Claim 5, wherein said metabolite concentration changes are displayed as a topographic image.

7. The living body light measurement system according to Claim 2, wherein said calculation part addition-averages metabolite concentration changes in trial periods where said metabolite concentration does not exceed a predetermined threshold, and displays the addition average of said metabolite concentration changes.

8. The living body light measurement system according to Claim 7, comprising a setting part which sets said threshold.

9. The living body light measurement system according to Claim 7, wherein said display displays the addition average of said metabolite concentration changes as a topographic image.

10. The living body light measurement system according to Claim 1, comprising a setting part which sets a rest period time, a task period time, and the number of times that a task is given to said subject.

11. The living body light measurement system according to Claim 1, comprising a measurement device which measures the motion of said subject by a sensor with which said subject is fitted.

12. The living body light measurement system according to Claim 11, wherein said display displays said metabolite concentration change together with measurement data obtained by said measurement device, or displays said moving image and measurement data obtained by said measurement device, in synchronism.

13. An image display methodology, comprising the steps of:

illuminating a subject,
detecting light which has dispersed inside said subject,
calculating a metabolite concentration change of said detection subject from light which has dispersed inside said detection subject,
capturing a moving image of said subject under measurement, measuring said metabolite concentration change in a first trial period and a second trial period, said first trial period and said second trial period having a rest period wherein said subject is left at rest, and a task period wherein a task is given to said subject, and displaying the moving image in said first trial period and said second trial period effectively in synchronism, and

when said metabolite concentration change is addition-averaged every trial period, selecting the trial periods to be used for calculating the addition average of said metabolite concentration change.

14. The image display methodology according to Claim 13, comprising a step wherein the addition average of said metabolite concentration change is displayed.

15. The image display methodology according to Claim 13, comprising a step for setting whether said moving image is to be displayed or not to be displayed for every trial period.

16. A program used in a living body light measurement system which measures a metabolite concentration change of a subject in a first trial period and a second trial period,
said first trial period and said second trial period having a rest period wherein said subject is left at rest, and a task period wherein a task is given to said subject,
wherein said program performs a step which displays moving images in said first trial period and said second trial period effectively in synchronism on a computer.

17. The program according to Claim 16, wherein said metabolite concentration change and said moving image are displayed effectively in synchronism.

18. The program according to Claim 16, comprising the steps of:

addition-averaging said metabolite concentration changes in trial periods wherein said metabolite concentration changes do not exceed a predetermined threshold, and
displaying the addition average of said metabolite concentration change and said moving image effectively in synchronism.

19. The program according to Claim 18, wherein said threshold is a variable which can be set.

20. The program according to Claim 18, wherein the addition average of said metabolite concentration change is displayed as a topographic image.

# FIG. 1

# FIG. 2

FIG. 3

EP 1 598 008 A1

# FIG. 4

## FIG. 5

Blood volume
increase

decrease

## FIG. 6

R T R T R T R T R T R

30 sec | 15 sec

$t_1$  $t_2$  $t_3$  $t_4$  $t_5$  $t_6$  $t_7$  $t_8$  $t_9$  $t_{10}$  $t_{11}$  $t_{12}$

Time(t)

FIG. 7

# FIG. 8

FIG. 9

# FIG. 10

Time  0 mins 13 secs

## FIG. 11

```
┌─────────────────────────────────────────┐
│  Setting of T (task period), R (rest period), │
│  n (task frequency), m (threshold value)      │
└─────────────────────────────────────────┘
                    │
                    ▼
          ┌──────────────────┐
          │ Measurement start │
          └──────────────────┘
                    │
                    ▼
          ┌──────────────────┐
   ┌─────▶│   Rest (R sec)    │
   │      └──────────────────┘
   │                │
   │                ▼
   │      ┌──────────────────┐
   │      │   Task (T sec)    │
   │      └──────────────────┘
   │                │
   │                ▼
   │          ◇ n times ◇
   │  No    ◇ completed? ◇
   └────────◇          ◇
                    │ Yes
                    ▼
          ┌──────────────────┐
          │   Rest (R sec)    │
          └──────────────────┘
                    │
                    ▼
     ┌────────────────────────────┐
     │ Save measurement data, image data │
     └────────────────────────────┘
                    │
                    ▼
     ┌────────────────────────────────────┐
     │ Save measurement data, image data for each task │
     └────────────────────────────────────┘
                    │
                    ▼
          ◇ Measurement data for ◇   Yes
          ◇ this task exceeds m?  ◇──────────┐
                    │ No                      │
                    ▼                         ▼
     ┌──────────────────────────┐   ┌──────────────────────────┐
     │ Image data or measurement data for │   │ -Image data for this task not │
     │ each task displayed in synchronism │   │  displayed                    │
     └──────────────────────────┘   │ -Measurement data for this    │
                    │                 │  task excluded from           │
                    ▼                 │  synchronous addition         │
     ┌──────────────────────────┐   └──────────────────────────┘
     │ Tasks not used (or used) for       │
     │ synchronous addition selected      │
     └──────────────────────────┘
                    │
                    ▼
     ┌────────────────────────────────────┐
     │ Synchronous addition performed with measurement │
     │ data for tasks used for synchronous addition    │
     └────────────────────────────────────┘
                    │
                    ▼
     ┌────────────────────────────────────┐
     │ Addition-averaged measurement data displayed │
     └────────────────────────────────────┘
```

# FIG. 12

Threshold value | 0.2 mM mm/s

12-1

12-2

12-3

12-4

## FIG. 13

Time | 0 mins 13 secs

Blood volume increase

decrease

13-1

## FIG. 14

Time     0 mins 13 secs

14-2

14-1

Blood volume
increase

decrease

FIG. 15

FIG. 16

FIG. 17

# FIG. 18

(A)

18-1

Distance
(mm)

100

0

0        10        20        30
Time (sec)

(B)

18-2

Velocity
(m/s)

2

0

-3

0        10        20        30
Time (sec)

# FIG. 19

(A)

19-1

Distance
(mm)

100

0

0              1              2              3
Time (sec)

(B)

19-2

Velocity
(m/s)

2

0

-3

T1

T2

0              1              2              3
Time (sec)

## FIG. 20

(A)

20-1

T1
(sec)

Tapping frequency

(B)

20-2

T2
(sec)

Tapping frequency

FIG. 21

# FIG. 22

| | | |
|---|---|---|
| Save location | Image folder ▽ | |

☐ Full image 1
☐ Image 1 (Task 1)
☐ Image 1 (Task 2)
☐ Image 1 (Task 3)
☐ Image 1 (Task 4)
☐ Image 1 (Task 5)

| | | |
|---|---|---|
| File name | Image 1(Task 1) ▽ | Save (s) |
| File type | Image ▽ | Cancel |

30

**European Patent Office**

# EUROPEAN SEARCH REPORT

Application Number

EP 05 01 0796

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WOLF, WOLF,CHOI,GUPTA,SAFONOVA,PAUNESCU,MICHALOS ,GRATTON,: "Functional Frequency-Domain Near-Infrared Spectroscopy Detects Fast Neuronal Signal in the Motor Cortex" NEUROIMAGE, vol. 17, no. 4, December 2002 (2002-12), pages 1868-1875, XP002339936 * page 1868 - page 1870 * * figure 1 * ----- | 1-20 | A61B5/11 A61B5/145 |
| Y | KURATA K, TSUJI T, NARAKI S, SEINO M, ABE Y.: "Activation of the dorsal premotor cortex and pre-supplementary motor area of humans during an auditory conditional motor task." J NEUROPHYSIOL., vol. 84, no. 3, September 2000 (2000-09), pages 1667-1672, XP002339937 * page 1667 - page 1668 * ----- | 1-20 | |
| A | MARIA ANGELA FRANCESCHINI, DAVID A. BOAS: "Noninvasive measurement of neuronal activity with near-infrared optical imaging" NEUROIMAGE, vol. 21, no. 1, January 2004 (2004-01), pages 372-386, XP002339938 * the whole document * ----- -/-- | 1-20 | TECHNICAL FIELDS SEARCHED (Int.Cl.7) A61B |

The present search report has been drawn up for all claims

| Place of search | Date of completion of the search | Examiner |
|---|---|---|
| The Hague | 9 August 2005 | Dydenko, I |

**European Patent Office**

**EUROPEAN SEARCH REPORT**

Application Number

EP 05 01 0796

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WINSTEIN CJ, GRAFTON ST, POHL PS.: "Motor Task Difficulty and Brain Activity: Investigation of Goal-Directed Reciprocal Aiming Using Positron Emission Tomography" J NEUROPHYSIOL, vol. 77, no. 3, March 1997 (1997-03), pages 1581-1594, XP002339939 * page 1582 - page 1583 * * figure 1 * ----- | 1-20 | |
| A | MAKI A ET AL: "SPATIAL AND TEMPORAL ANALYSIS OF HUMAN MOTOR ACTIVITY USING NONINVASIVE NIR TOPOGRAPHY" MEDICAL PHYSICS, AMERICAN INSTITUTE OF PHYSICS. NEW YORK, US, vol. 22, no. 12, 1 December 1995 (1995-12-01), pages 1997-2005, XP000550635 ISSN: 0094-2405 * page 2000 * * figure 7 * ----- | 3,6,9,20 | |
| A | EP 1 402 820 A (HITACHI MEDICAL CORPORATION) 31 March 2004 (2004-03-31) * page 4, paragraph 19 * * page 5, paragraph 28 - page 6, paragraph 36 * ----- | 5,7,8, 10,14, 18,19 | TECHNICAL FIELDS SEARCHED (Int.Cl.7) |

The present search report has been drawn up for all claims

| Place of search | Date of completion of the search | Examiner |
|---|---|---|
| The Hague | 9 August 2005 | Dydenko, I |

EPO FORM 1503 03.82 (P04C01)

**ANNEX TO THE EUROPEAN SEARCH REPORT
ON EUROPEAN PATENT APPLICATION NO.**                    EP 05 01 0796

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2005

| Patent document cited in search report | | Publication date | Patent family member(s) | | Publication date |
|---|---|---|---|---|---|
| EP 1402820 | A | 31-03-2004 | JP | 2003010188 A | 14-01-2003 |
| | | | EP | 1402820 A1 | 31-03-2004 |
| | | | US | 2004242979 A1 | 02-12-2004 |
| | | | CN | 1520274 A | 11-08-2004 |
| | | | WO | 03002004 A1 | 09-01-2003 |

For more details about this annex : see Official Journal of the European Patent Office, No. 12/82